# EUROPEAN PATENT SPECIFICATION

(11) **EP 1 116 714 B1**
(45) Date of publication and mention of the grant of the patent: **20.08.2003**
(21) Application number: 01810001.6
(22) Date of filing: 03.01.2001
(51) Int. Cl.: C07C 323/16, C07C 319/14, C07C 319/28

(54) **Improved process for the preparation of mercaptomethylphenols**
Verfahren zur Herstellung von Merkaptomethylphenolen
Procédé pour la préparation de mercaptophénylphénols

(30) Priority: 10.01.2000 EP 00810020
(43) Date of publication of application: 18.07.2001
(73) Proprietor: Ciba Specialty Chemicals Holding Inc., 4057 Basel (CH); Ciba Specialty Chemicals S.p.A., 40037 Sasso Marconi (BO) (IT)
(72) Inventor: Pizzoli, Fabio, 40137 Bologna (IT); Luisoli, Reto, 4434 Hölstein (CH); Knobloch, Gerrit, 4312 Magden (CH); Meier, Hans-Rudolf, 1700 Fribourg (CH)

(56) References cited:
- US-A- 4 874 885
- DATABASE CHEMABS [Online] CHEMICAL ABSTRACTS SERVICE, COLUMBUS, OHIO, US; STN, CAPLUS accession no. 1990:425944, XP002159315 & JP 02 042055 A (NISSO PETROCHEMICAL INDUSTRIES CO LTD ET AL) 13 February 1990 (1990-02-13)

## Description

The present invention relates to a new improved process for the preparation of mercaptomethylphenols from phenols by reaction with formaldehyde and mercaptans and which process comprises treating afterwards the reaction product with a reducing agent. The mercaptomethylphenols are valuable antioxidants for plastics, elastomers mineral oils and synthetic lubricants.

U.S. 4,857,572 discloses mercaptomethylphenols as antioxidants for plastics, elastomers, tackifying resins, mineral oils and lubricants.

U.S. 4,874,885 discloses a process for the preparation of mercaptomethylphenols from phenols by reaction with formaldehyde and mercaptans in the presence of mono-, di- or trimethylamine or mono- or diethylamine.

U.S. 5,276,258 discloses that the stabilising properties of mercaptomethylphenols in elastomers can be improved by the additional use of an epoxidised fatty acid or fatty acid ester.

The mercaptomethylphenols prepared according to the known processes have the disadvantages that they for example possess an undesired smell and discolour in the presence of lithium catalysts or alkaline medium mainly because of the presence of undesired by-products or impurities.

The goal of the instant invention was therefore to find an improved process for the synthesis of mercaptomethylphenols with reduced smell, with reduced discoloration in the presence of for example lithium catalysts and alkaline medium, and with excellent storage stability.

Surprisingly, it has now been found that treating the crude mercaptomethylphenols prepared according to the process disclosed in U.S. 4,874,885 afterwards with a reducing agent improves the stabilizing qualitiy of the mercaptomethylphenols.

The present invention therefore relates to an improved process for the preparation of a compound of the formula I 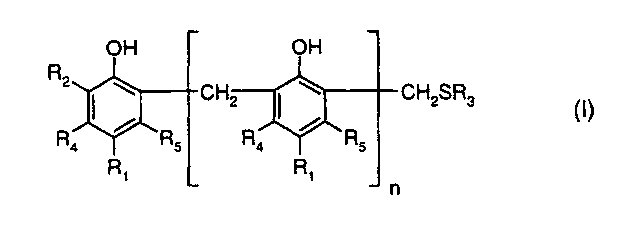 wherein
n is 0 or 1,
R₁ is C₁-C₁₂alkyl or -CH₂SR₃,
R₂ is C₁-C₁₂alkyl, C₇-C₉phenylalkyl, C₇-C₉phenylalkyl substituted on the phenyl radical by from 1 to 3 C₁-C₄alkyl groups; or -CH₂SR₃,
R₃ is C₆-C₁₈alkyl, phenyl or benzyl,
R₄ is hydrogen or methyl,
R₅ is hydrogen or methyl, with the proviso that R₄ and R₅ are not simultaneously methyl, by reacting a compound of the formula II 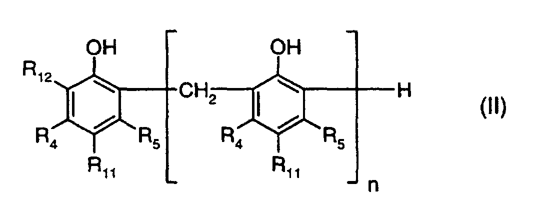 wherein n, R₄ and R₅ are as previously defined,
R₁₁ is hydrogen or C₁-C₁₂alkyl; and
R₁₂ is hydrogen, C₁-C₁₂alkyl, C₇-C₉phenylalkyl, C₇-C₉phenylalkyl substituted on the phenyl radical by from 1 to 3 C₁-C₄alkyl groups, with formaldehyde or a compound that liberates formaldehyde under the reaction conditions and with at least one compound of the formula III

R₃SH (III)

wherein R₃ is as previously defined, in the presence of a base, said base being mono- or dimethylamine or mono- or diethylamine, which process comprises treating afterwards the reaction product with a reducing agent.

Alkyl having up to 18 carbon atoms is a branched or unbranched radical, for example methyl, ethyl, propyl, isopropyl, n-butyl, sec-butyl, isobutyl, tert-butyl, 2-ethylbutyl, n-pentyl, isopentyl, 1-methylpentyl, 1,3-dimethylbutyl, n-hexyl, 1-methylhexyl, n-heptyl, isoheptyl, 1,1,3,3-tetramethylbutyl, 1-methylheptyl, 3-methylheptyl, n-octyl, tert-octyl, 2-ethylhexyl, 1,1,3-trimethylhexyl, 1,1,3,3-tetramethylpentyl, nonyl, decyl, undecyl, 1-methylundecyl, dodecyl, tridecyl, 1,1,3,3,5,5-hexamethylhexyl, tetradecyl, pentadecyl, hexadecyl, heptadecyl or octadecyl. One of the preferred definitions for R₁,R₂, R₁₁ and R₁₂ is C₁-C₁₂alkyl, especially preferred is C₁-C₄alkyl, for example methyl. A preferred definition for R₃ is C₈-C₁₂alkyl, for example, n-octyl or n-dodecyl.

C₇-C₉Phenylalkyl unsubstituted or substituted on the phenyl radical by from 1 to 3 C₁-C₄alkyl groups is, for example, benzyl, α-methylbenzyl, α,α-dimethylbenzyl, 2-phenylethyl, 2-methylbenzyl, 3-methylbenzyl, 4-methylbenzyl, 2,4-dimethylbenzyl, 2,6-dimethylbenzyl or 4-tert-butylbenzyl. Preference is given to benzyl, α-methylbenzyl or α,α-dimethylbenzyl.

Of interest is a process for the preparation of a compound of formula I wherein,
n is 0 or 1,
R₁ is C₁-C₄alkyl or -CH₂SR₃,
R₂ is C₁-C₄alkyl, C₇-C₉phenylalkyl, C₇-C₉phenylalkyl substituted on the phenyl radical by a methyl group,
R₃ is C₆-C₁₂alkyl,
R₄ is hydrogen or methyl,
R₅ is hydrogen,
R₁₁ is hydrogen or C₁-C₄alkyl; and
R₁₂ is C₁-C₄alkyl, C₇-C₉phenylalkyl, C₇-C₉phenylalkyl substituted on the phenyl radical by a methyl group.

Likewise of interest is a process for the preparation of a compound of formula I wherein
n is 0,
R₁ is -CH₂SR₃,
R₂ is C₁-C₄alkyl,
R₃ is C₈-C₁₂alkyl,
R₄ is hydrogen,
R₅ is hydrogen,
R₁₁ is hydrogen; and
R₁₂ C₁-C₄alkyl.

Of special interest is a process for the preparation of 2,4-bis(n-octylthiomethyl)-6-methylphenol [Irganox 1520 (RTM), Ciba Specialty Chemicals] and of 2,4-bis(n-dodecylthiomethyl)-6-methylphenol.

The reaction of the compound of the formula II with formaldehyde and a compound of the formula III is carried out in the presence of mono- or dimethylamine or mono- or diethylamine as base. It is preferred to use dimethylamine as base. The base may be for example in the form of a 10 - 35 % by weight solution in ethanol, methanol or another lower alcohol or in pure form. Dimethylamine can also be used in gaseous form.

The base can be used for example in an amount of 1 to 50 mol % and, most preferably, 5 - 20 mol %, based on the compound of the formula III.

The reaction of the compound of the formula II with formaldehyde and a compound of the formula III can be carried out in the presence of a solvent.

Examples of suitable solvents are alcohols of 1 to 6 carbon atoms, for example methanol, ethanol, propanol, butanol, pentanol or hexanol. However, it is also possible to use diols, polyols and ethers thereof, for example glycol, glycerol and polyethylene glycol. The reaction can be carried out in a polar aprotic solvent such as dimethylformamide or dimethylsulfoxide, or a high-boiling aromatic or aliphatic hydrocarbon or chlorinated hydrocarbon such as toluene, ligroin or chlorobenzene. The preferred solvent is dimethylformamide, which is diluted with one of the above mentioned lower alcohols or chlorinated hydrocarbons. It is preferred, however, to carry out the process in the absence of a solvent.

The reaction of the compound of the formula II with formaldehyde and a compound of the formula III can conveniently be carried out in the temperature range from 80°C to 160°C, preferably from 90°C to 150°C, and most preferably, from 90°C to 130°C, and at normal pressure or under pressure (e.g. from 0.01 to 5 bar). In the absence of a solvent the reaction is preferably carried out under overpressure.

Depending on the specific compound of the formula II and compound of the formula III employed, the reaction times may vary and are for example from 1 to 24 hours and, preferably, from 1 to 6 hours. The reaction mixture is conveniently heated in a nitrogen atmosphere under reflux.

After cooling to room temperature, the reaction mixture is worked up by conventional separating and purifying methods.

Most of the compounds of the formula I, II and III are known and some are commercially available or can be prepared by known methods.

Formaldehyde or a compound that liberates formaldehyde under the reaction conditions, for example para-formaldehyde or hexamethylenetetramine, is used for the reaction. It is preferred to use formaldehyde, but para-formaldehyde is particularly preferred.

A preferred reducing agent is a hydride or hydrogen with a catalyst.

Preferred catalysts for hydrogenation are for example Pt, Pd, Rh, Ru, Ni like for example Raney-nickel, or Cu-Cr systems. The metals are supported on inert supports like for example carbon, alumina, barium sulfate. Especially preferred catalysts are Pt, Ru, Ni and Cu-Cr.

Of special interest is a process for the preparation of a compound of formula I wherein the reducing agent is a hydride.

Hydrides of special interest are for example sodium hydride, potassium hydride, calcium hydride, lithium aluminium hydride, aluminium hydride, sodium cyanoborohydride, sodium borohydride or diisobutylaluminium hydride. Preferred hydrides are sodium borohydride, sodium cyanoborohydride and diisobutylaluminium hydride.

Of very special interest is a process for the preparation of a compound of formula I wherein the reducing agent is sodium borohydride.

Advantageously, the reducing agent is used in an amount of from 0.02 to 10 % by weight, especially from 0.02 to 1 % by weight, e.g. from 0.07 to 0.5 % by weight, based on the weight of the compound of the formula I.

The reaction step with a reducing agent can conveniently be carried out in the temperature range from 20°C to 200°C, preferably from 40°C to 150°C, and most preferably, from 60°C to 100°C.

The reaction step with a reducing agent can be carried out in the presence of a solvent. Examples of suitable solvents are the same as mentioned above for the reaction of the compound of the formula II with formaldehyde and a compound of the formula III. It is preferred, however, to carry out the reduction step with a reducing agent in the absence of a solvent.

After cooling to room temperature, the reaction mixture is worked up by conventional separating and purifying methods.

The compounds of the formula I prepared by the process of this invention can be used as stabilisers for protecting organic materials from damage by the action of oxygen, heat, light or high-energy radiation. The preferred utility of these compounds is as antioxidants in organic polymers and in elastomers, or in mineral oils or synthetic lubricants.

The stabilisers are normally added to the organic materials in a concentration of 0.01 to 10 % by weight, preferably 0.05 to 5.0 % by weight, most preferably 0.1 to 2.0 % by weight based on the organic material to be stabilised.

Incorporation of the compounds of the formula I can be effected, for example, by blending them with the material to be stabilised together with further optional additives by methods conventionally employed in the art, before or during the manufacture of articles shaped from said polymer, or also by applying the dissolved or dispersed compound to the polymer, with or without subsequent evaporation of the solvent. The compounds of the formula I may also be added to the materials to be stabilised in the form of a masterbatch which contains said compounds, for example, in a concentration of 2.5 to 25 % by weight. In the case of crosslinkable polyethylene, the compounds are added prior to crosslinking.

In practice, the compounds of the formula I are added together with other stabilisers.

The compounds of the formula I are added to a rubber cement or latex preferably after polymerisation but prior to coagulation.

Lubricant formulations may also contain further additives which are added to improve certain use properties, for example further antioxidants, metal deactivators, rust inhibitors, viscosity index improvers, pour-point depressors, dispersants/surfactants and antiwear additives.

The following Examples illustrate the invention further. Parts or percentages relate to weight.

### Example 1: Preparation of 2,4-bis(n-octylthiomethyl)-6-methylphenol.

a) In a 1 liter reactor 84.2 g (2.81 mol) of para-formaldehyde, 134.9 g (1.25 mol) of o-cresol and 369.3 g (2.52 mol) of octhanethiol are charged. Then 9.9 g (0.22 mol) of dimethylamine is added. The temperature is then increased to 130°C within one hour. The temperature is kept at 130° C for 3.5 hours. The volatile constituents are then removed at a bath temperature of 90 - 95°C under reduced pressure to give the crude 2,4-bis(n-octylthiomethyl)-6-methylphenol.
b) In a 500 ml flask, 390 g (0.92 mol) of crude 2,4-bis(n-octylthiomethyl)-6-methylphenol prepared according to Example 1a is mixed with 3.25 g of a caustic solution containing 12 % of sodium borohydride, corresponding to 0.39g (0.01 mol) of sodium borohydride. The mixture is heated at 90 °C for 7 hours. Afterwards the reaction mixture is extracted with acetic acid and water. The volatile constituents are then removed at a bath temperature of 90 - 95°C under reduced pressure to give 380 g of 2,4-bis(n-octylthiomethyl)-6-methylphenol.

### Example 2: Preparation of 2,4-bis(n-octylthiomethyl)-6-methylphenol.

In a 500 ml flask, 410 g (0,97 mol) of crude 2,4-bis(n-octylthiomethyl)-6-methylphenol prepared according to Example 1a is mixed with 17 g of a caustic solution containing 12 % of sodium borohydride; corresponding to 2.04 g (0.05mol) of sodium borohydride. The mixture is heated at 90°C for 7 hours. Afterwards, the reaction mixture is extracted with acetic acid and water. The volatile constituents are then removed at a bath temperature of 90 - 95°C under reduced pressure to give 400 g of 2,4-bis(n-octylthiomethyl)-6-methylphenol.

### Example 3: Preparation of 2,4-bis(n-octylthiomethyl)-6-methylphenol.

In a 500 ml flask, 390 g (0,92 mol) of crude 2,4-bis(n-octylthiomethyl)-6-methylphenol prepared according to Example 1a is mixed with 1 g (0.026 mol) sodium borohydride. The mixture is heated at 90°C for 7 hours. Afterwards, the reaction mixture is filtered and extracted with acetic acid and water. The volatile constituents are then removed at a bath temperature of 90 - 95°C under reduced pressure to give 380 g of 2,4-bis(n-octylthiomethyl)-6-methylphenol.

### Example 4: Discoloration of mercaptomethylphenols in alkaline environment.

Various rubbers are polymerised in aliphatic organic solvents using n-butyl-lithium as catalyst/initiator (anionic polymerisation). After decomposition of the catalyst by means of water, lithium-hydroxide is formed as transition product. Thus the rubber cement is very alkaline as well as the water of the rubber coagulation system. Phenolic antioxidants are partly transformed under such conditions into the corresponding phenolates. Some of these phenolates or by-product phenolates have a yellow or orange colour. This discoloration leads to a yellow coloured rubber instead of a water white colourless rubber.

The tendency to discolour can be determined by a direct reaction of the phenolic antioxidant with the polymerisation initiator butyl-lithium. For this purpose 0.1 mmol n-butyl-lithium is added in form of a 10% solution in n-hexane to an equivalent amount of 2,4-bis(n-octylthiomethyl)-6-methylphenol dissolved in 100 ml n-hexane.

A sodium methylate treatment can be used as alternative method in order to avoid the use of butyl-lithium. 0.5 g of 2,4-bis(n-octylthiomethyl)-6-methylphenol according to Examples 1a, 1b, 2 and 3 were dissolved in 50 ml methanol; 0.5 g of a 10 % solution of NaOCH₃ in methanol is added. The transmission of these solutions is measured at 300-500 nm using the UV-visible recording spectrophotometer Shimadzu UV-2100. The higher the transmission in % the better is the quality of the stabiliser. The results are summarised in Table 1. The discoloration can also be determined by measuring the yellowness Index. In this case, the sodium methoxide method was used with a quantity of 2.5 g 2,4-bis(n-octylthiomethyl)-6-methylphenol instead of 0.5 g. The yellowness index (YI) of the samples is determined in accordance with ASTM D 1925-70. Low YI values denote little discolouration, high YI values severe discoloration of the samples. The less discolouration, the more effective the stabiliser. The results are compiled in Table 1.

**Table 1:**

| Examples | Stabiliser prepared according to | Transmission in % at 425 nm | Yellowness Index |
|---|---|---|---|
| Example 4a^{a)} | Example 1a | 95.5 | 4.2 |
| Example 4b^{b)} | Example 1b | 98.6 | 2.8 |
| Example 4c^{b)} | Example 2 | 98.5 | 2.8 |
| Example 4d^{b)} | Example 3 | 98.5 | 2.8 |

| | | | |
|---|---|---|---|
| a) Comparison Example. | | | |
| b) Examples of this invention. | | | |

## Claims

1. An improved process for the preparation of a compound of the formula I 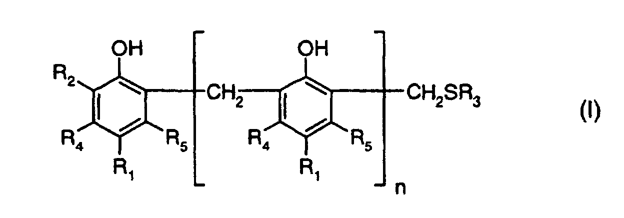 wherein
n is 0 or 1,
R₁ is C₁-C₁₂alkyl or -CH₂SR₃,
R₂ is C₁-C₁₂alkyl, C₇-C₉phenylalkyl, C₇-C₉phenylalkyl substituted on the phenyl radical by from 1 to 3 C₁-C₄alkyl groups; or -CH₂SR₃,
R₃ is C₆-C₁₈alkyl, phenyl or benzyl,
R₄ is hydrogen or methyl,
R₅ is hydrogen or methyl, with the proviso that R₄ and R₅ are not simultaneously methyl, by reacting a compound of the formula II 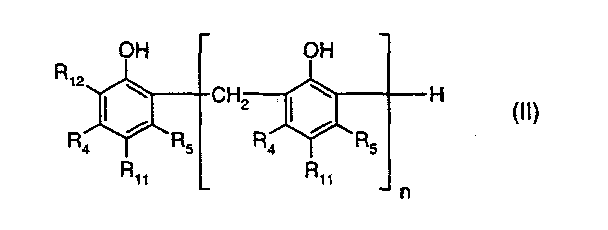 wherein n, R₄ and R₅ are as previously defined,
R₁₁ is hydrogen or C₁-C₁₂alkyl; and
R₁₂ is hydrogen, C₁-C₁₂alkyl, C₇-C₉phenylalkyl, C₇-C₉phenylalkyl substituted on the phenyl radical by from 1 to 3 C₁-C₄alkyl groups, with formaldehyde or a compound that liberates formaldehyde under the reaction conditions and with at least one compound of the formula III
R₃SH (III)
wherein R₃ is as previously defined, in the presence of a base, said base being mono- or dimethylamine or mono- or diethylamine, which process comprises treating afterwards the reaction product with a reducing agent.

2. A process according to claim 1, wherein
n is 0 or 1,
R₁ is C₁-C₄alkyl or -CH₂SR₃,
R₂ is C₁-C₄alkyl, C₇-C₉phenylalkyl, C₇-C₉phenylalkyl substituted on the phenyl radical by a methyl group,
R₃ is C₆-C₁₂alkyl,
R₄ is hydrogen or methyl,
R₅ is hydrogen,
R₁₁ is hydrogen or C₁-C₄alkyl; and
R₁₂ is C₁-C₄alkyl, C₇-C₉phenylalkyl, C₇-C₉phenylalkyl substituted on the phenyl radical by a methyl group.

3. A process according to claim 1, wherein
n is 0,
R₁ is -CH₂SR₃,
R₂ is C₁-C₄alkyl,
R₃ is C₈-C₁₂alkyl,
R₄ is hydrogen,
R₅ is hydrogen,
R₁₁ is hydrogen; and
R₁₂ C₁-C₄alkyl.

4. A process according to claim 1, wherein the base is dimethylamine.

5. A process according to claim 1, wherein 1 to 50 mol % of base is used, based on the compound of the formula III.

6. A process according to claim 1, wherein the reducing agent is a hydride.

7. A process according to claim 6, wherein the hydride is sodium borohydride.

8. A process according to claim 1, wherein the amount of reducing agent is 0.02 to 10 % by weight based on the weight of the compound of the formula I.

9. A process according to claim 1, wherein the reaction is carried out in the absence of a solvent.

## Patentansprüche

1. Verbessertes Verfahren zur Herstellung einer Verbindung der Formel I 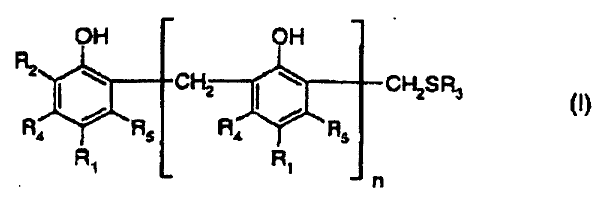 worin n 0 oder 1 ist,
R₁ C₁-C₁₂-Alkyl oder -CH₂SR₃ darstellt,
R₂ C₁-C₁₂-Alkyl, C₇-C₉-Phenylalkyl, C₇-C₉-Phenylalkyl, substituiert an dem Phenylrest mit 1 bis 3 C₁-C₄-Alkylgruppen, oder -CH₂SR₃ darstellt,
R₃ C₆-C₁₈-Alkyl, Phenyl oder Benzyl darstellt,
R₄ Wasserstoff oder Methyl darstellt,
R₅ Wasserstoff oder Methyl darstellt, mit der Maßgabe, dass R₄ und R₅ nicht gleichzeitig Methyl darstellen, durch Umsetzen einer Verbindung der Formel II 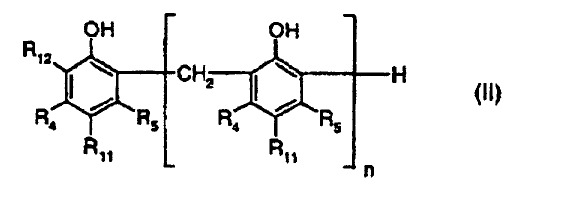 worin n, R₄ und R₅ wie vorstehend definiert sind,
R₁₁ Wasserstoff oder C₁-C₁₂-Alkyl darstellt; und
R₁₂ Wasserstoff, C₁-C₁₂-Alkyl, C₇-C₉-Phenylalkyl, C₇-C₉-Phenylalkyl, substituiert an dem Phenylrest mit 1 bis 3 C₁-C₄-Alkylgruppen, darstellt, mit Formaldehyd oder einer Verbindung, die Formaldehyd unter den Reaktionsbedingungen freisetzt und mit mindestens einer Verbindung der Formel III
R₃SH (III),
worin R₃ wie vorstehend definiert ist,
in Gegenwart einer Base, wobei die Base Mono- oder Dimethylamin oder Mono- oder Diethylamin darstellt, wobei das Verfahren anschließend Behandeln des Reaktionsproduktes mit einem Reduktionsmittel umfasst.

2. Verfahren nach Anspruch 1, wobei
n 0 oder 1 ist,
R₁ C₁-C₄-Alkyl oder -CH₂SR₃ darstellt,
R₂ C₁-C₄-Alkyl, C₇-C₉-Phenylalkyl, C₇-C₉ Phenylalkyl, substituiert an dem Phenylrest mit einer Methylgruppe, darstellt,
R₃ C₆-C₁₂-Alkyl darstellt,
R₄ Wasserstoff oder Methyl darstellt,
R₅ Wasserstoff darstellt,
R₁₁ Wasserstoff oder C₁-C₄-Alkyl darstellt und
R₁₂ C₁-C₄-Alkyl, C₇-C₉-Phenylalkyl, C₇-C₉ Phenylalkyl, substituiert an dem Phenylrest mit einer Methylgruppe, darstellt.

3. Verfahren nach Anspruch 1, wobei
n 0 ist,
R₁ -CH₂SR₃ darstellt,
R₂ C₁-C₄-Alkyl darstellt,
R₃ C₈-C₁₂-Alkyl darstellt,
R₄ Wasserstoff darstellt,
R₅ Wasserstoff darstellt,
R₁₁ Wasserstoff darstellt und
R₁₂ C₁-C₄-Alkyl darstellt.

4. Verfahren nach Anspruch 1, wobei die Base Dimethylamin darstellt.

5. Verfahren nach Anspruch 1, wobei 1 bis 50 Molprozent Base, bezogen auf die Verbindung der Formel III, verwendet werden.

6. Verfahren nach Anspruch 1, wobei das Reduktionsmittel ein Hydrid darstellt.

7. Verfahren nach Anspruch 6, wobei das Hydrid Natriumborhydrid darstellt.

8. Verfahren nach Anspruch 1, wobei die Menge an Reduktionsmittel 0,02 bis 10 Gewichtsprozent, bezogen auf das Gewicht der Verbindung der Formel I, ist.

9. Verfahren nach Anspruch 1, wobei die Reaktion in Abwesenheit eines Lösungsmittels ausgeführt wird.

## Revendications

1. Procédé perfectionné pour la préparation d'un composé de formule I 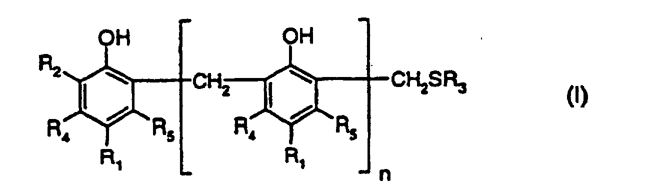 dans laquelle
n est 0 ou 1,
R₁ est un groupe alkyle en C₁-C₁₂ ou -CH₂SR₃,
R₂ est un groupe alkyle en C₁-C₁₂, phénylalkyle en C₇-C₉, phénylalkyle en C₇-C₉ substitué sur le radical phényle par 1 à 3 groupes alkyle en C₁-C₄ ; ou -CH₂SR₃,
R₃ est un groupe alkyle en C₆-C₁₈, phényle ou benzyle,
R₄ est l'hydrogène ou un groupe méthyle,
R₅ est l'hydrogène ou un groupe méthyle, à condition que R₄ et R₅ ne soient pas simultanément un groupe méthyle,
par réaction d'un composé de formule II 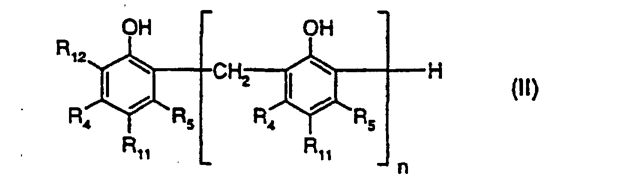 dans laquelle
n, R₄ et R₅ sont tels que définis précédemment,
R₁₁ est l'hydrogène ou un groupe alkyle en C₁-C₁₂, et
R₁₂ est l'hydrogène, un groupe alkyle en C₁-C₁₂, phénylalkyle en C₇-C₉, phénylalkyle en C₇-C₉ substitué sur le radical phényle par 1 à 3 groupes alkyle en C₁-C₄,
avec le formaldéhyde ou un composé qui libère du formaldéhyde dans les conditions réactionnelles, et avec au moins un composé de formule III
R₃SH (III)
dans laquelle R₃ est tel que défini précédemment, en présence d'une base, ladite base étant la mono- ou diméthylamine ou la mono- ou diéthylamine, lequel procédé comprend un traitement postérieur du produit réactionnel avec un agent réducteur.

2. Procédé selon la revendication 1, dans lequel
n est 0 ou 1,
R₁ est un groupe alkyle en C₁-C₄ ou -CH₂SR₃,
R₂ est un groupe alkyle en C₁-C₄, phénylalkyle en C₇-C₉, phénylalkyle en C₇-C₉ substitué sur le radical phényle par un groupe méthyle,
R₃ est un groupe alkyle en C₆-C₁₂,
R₄ est l'hydrog ou un groupe méthyle,
R₅ est l'hydrogène,
R₁₁ est l'hydrogène ou un groupe alkyle en C₁-C₄, et
R₁₂ est un groupe alkyle en C₁-C₄, phénylalkyle en C₇-C₉, phénylalkyle en C₇-C₉ substitué sur le radical phényle par un groupe méthyle.

3. Procédé selon la revendication 1, dans lequel
n est 0,
R₁ est -CH₂SR₃,
R₂ est un groupe alkyle en C₁-C₄,
R₃ est un groupe alkyle en C₈-C₁₂,
R₄ est l'hydrogène,
R₅ est l'hydrogène,
R₁₁ est l'hydrogène, et
R₁₂ est un groupe alkyle en C₁-C₄.

4. Procédé selon la revendication 1, dans lequel la base est la diméthylamine.

5. Procédé selon la revendication 1, dans lequel on utilise 1 à 50 mol % de base par rapport au composé de formule III.

6. Procédé selon la revendication 1, dans lequel l'agent réducteur est un hydrure.

7. Procédé selon la revendication 6, dans lequel l'hydrure est le borohydrure de sodium.

8. Procédé selon la revendication 1, dans lequel la quantité d'agent réducteur est de 0,02 à 10 % en poids par rapport au poids du composé de formule I.

9. Procédé selon la revendication 1, dans lequel la réaction est conduite en l'absence de solvant.
